# EUROPEAN PATENT APPLICATION

(11) **EP 2 295 028 A1**
(43) Date of publication of application: **16.03.2011**
(21) Application number: 09728262.8
(22) Date of filing: 31.03.2009
(51) Int. Cl.: A61K 8/46, A61K 8/49, A61K 31/17, A61K 31/4184, A61K 36/00, A61K 36/18, A61K 36/23, A61K 36/73, A61K 36/75, A61K 36/81, A61K 36/896, A61K 45/00, A61P 17/00, A61Q 19/08

(54) **PREPARATION FOR PREVENTING OR AMELIORATING WRINKLES, TO BE TAKEN ORALLY, THROUGH INJECTION, OR THROUGH EXTERNAL APPLICATION TO SKIN, AND COSMETIC METHOD**

(30) Priority: 31.03.2008 WO PCT/JP2008/056421; 24.10.2008 JP 2008274944
(71) Applicant: Shiseido Company, Ltd., Chuo-ku Tokyo 104-8010 (JP)
(72) Inventor: IRIYAMA, Shunsuke, Yokohama-shi Kanagawa 224-8558 (JP); MATSUNAGA, Yukiko, Yokohama-shi Kanagawa 236-8643 (JP); AMANO, Satoshi, Yokohama-shi Kanagawa 224-8558 (JP); UMISHIO, Kenichi, Yokohama-shi Kanagawa 224-8558 (JP); KUSAKARI, Ken, Yokohama-shi Kanagawa 224-8558 (JP); HIRUMA, Takuya, Yokohama-shi Kanagawa 224-8558 (JP)
(74) Representative: Ulmann, Catherine Claire
(86) International application number: PCT/JP2009/056717
(87) International publication number: WO 2009/123215

(57) **Abstract**

Wrinkles are improved by applying an oral preparation, injection preparation or external skin preparation having a substance that controls heparanase as an active ingredient thereof.

## Description

### TECHNICAL FIELD

The present invention relates to an oral preparation, injection preparation, external skin preparation and cosmetic method for preventing or improving wrinkles, and to a method for evaluating anti-wrinkle effects.

### BACKGROUND ART

Although the number of wrinkles increases as a phenomenon of skin aging as one grows older, interest in prevention and improvement of wrinkles is becoming extremely high from the viewpoint of aesthetics and the like particularly among women. Wrinkles are broadly classified into large wrinkles, fine wrinkles and crepe paper wrinkles depending on the location where they occur, the mechanism of their occurrence and the like. Large wrinkles are deep wrinkles that mainly occur on the face and back of the neck due to photoaging, fine wrinkles are comparatively shallow wrinkles that occur around the eyes and mouth, and crepe paper wrinkles are crease-like wrinkles that occur at locations not exposed to sun such as on the abdomens of elderly persons.

Fine wrinkles in particular have been reported to demonstrate high values for wrinkle surface area ratio in persons having low horny layer moisture content (see Ugawa, et al., Fragrance Journal, 1992 (11), 29-42 (Non-Patent Document 1)), and fine wrinkles have been determined to be aggravated by decreases in horny layer moisture content caused by skin chapping and drying. The skin of modern women is subjected to extremely dry environments even during the summer months due to the proliferation of air-conditioners, and the number of women who are concerned about fine wrinkles or have an interest in fine wrinkles is increasing.

Although methods such as enhancing moisture retention of skin by applying moisturizing components such as glycerin, natural moisturizing factor (NMF)-related components or collagen derivatives and the like to the skin to restore decreased horny layer moisture content have been previously proposed to improve wrinkles, pharmaceutical preparations having fine wrinkle improving effects superior to that of moisturizing action have yet to be developed.

In recent years however, biochemical changes in the skin are being elucidated through the development of fine wrinkle models (see Matsunaga, et al., British Journal of Dermatology, 2007, May, 156(5): 884-91 (Non-Patent Document 2)). As a result thereof, activation of A Disintegrin and Metalloproteinase (ADAM), and its accompanying release of growth factors bound to the membrane of epidermal cells, such as heparin-binding epidermal growth factor-like growth factor (HB-EGF) or amphiregulin and the like, has been determined to promote the formation of fine wrinkles by causing epidermal hyperplasia and dermal hyperplasia. Compositions for inhibiting skin aging incorporating ADAM activity inhibitors such as TAPI-1 or 4-methoxybenzohydroxamic acid have been proposed to prevent the formation of small wrinkles (see Japanese Unexamined Patent Publication No. 2006-137744 (Patent Document 1), Japanese Unexamined Patent Publication No. 2007-119444 (Patent Document 2)).

However, detailed studies have yet to be adequately conducted relating to various changes at the biochemical level, including those at the protein level and gene level, that occur in the skin accompanying the formation of fine wrinkles, and there is a strong desire for an oral preparation, injection preparation, external skin preparation and cosmetic method capable of preventing and/or improving fine wrinkles in a more effective manner based on such detailed studies.

Patent Document 1: Japanese Unexamined Patent Publication No. 2006-137744
Patent Document 2: Japanese Unexamined Patent Publication No. 2007-119444
Patent Document 3: Published Japanese Translation of PCT Application No. 2003-502054
Non-Patent Document 1: Fragrance Journal, 1992 (11), 29-42
Non-Patent Document 2: British Journal of Dermatology, 2007, May, 156(5): 884-91
Non-Patent Document 3: Semin. Cancer Biol., 2002, 12 (2): 121-129
Non-Patent Document 4: Mol. Cancer Ther., 2004, 3(9): 1069-1077
Non-Patent Document 5: Bioorg. Med. Chem. Lett., 2006, (16): 409-412

### DISCLOSURE OF THE INVENTION

### Problems to be Solved by the Invention

In consideration of the aforementioned circumstances, an object of the present invention is to elucidate biochemical changes in the skin involved in the formation of fine wrinkles, specify substances that are able to inhibit those changes, and use those substances to provide an oral preparation, injection preparation, external skin preparation or cosmetic method that is able to more effectively prevent or improve wrinkles. Moreover, an object of the present invention is to provide a method for enabling anti-wrinkle effects of test substances to be evaluated more efficiently and easily by using inhibition of the aforementioned biochemical changes as an indicator.

### Means for Solving the Problems

As a result of biochemically analyzing the process of wrinkle formation induced in a fine wrinkle model, the inventors of the present invention found that expression of heparanase increases and that heparan sulfate chains are decomposed to perlecan, which is a basement membrane heparan sulfate proteoglycan, in the wrinkle formation process, and discovered that wrinkle formation can be inhibited by applying a substance that inhibits the activity of heparanase, and the invention is based on these findings.
Thus, the present application includes the following inventions:
(1) a cosmetic method for preventing or improving wrinkles, characterized by controlling the action of heparanase present in skin;
(2) a cosmetic method for preventing or improving wrinkles, characterized by applying a substance that inhibits gene expression as a method for controlling the action of heparanase present in skin;
(3) a cosmetic method for preventing or improving wrinkles, characterized by applying a substance that inhibits gene translation as a method for controlling the action of heparanase present in skin;
(4) a cosmetic method for preventing or improving wrinkles, characterized by applying a substance that inhibits enzyme activity as a method for controlling the action of heparanase present in skin;
(5) a cosmetic method for preventing or improving wrinkles, characterized by applying a substance that inhibits enzyme activation as a method for controlling the action of heparanase present in skin;
(6) a cosmetic method for preventing or improving wrinkles, characterized by administering a substance that inhibits the action of heparanase present in skin by a method such as oral administration, injection or external application;
(7) an orally administered pharmaceutical preparation for preventing or improving wrinkles, comprising a substance that controls the action of heparanase present in skin as an active ingredient thereof;
(8) an injection preparation for preventing or improving wrinkles, comprising a substance that controls the action of heparanase in skin as an active ingredient thereof;
(9) an external skin preparation for preventing or improving wrinkles, comprising a substance that controls the action of heparanase in skin as an active ingredient thereof;
(10) the orally administered pharmaceutical preparation, injection preparation or external skin preparation of any of (7) to (9), wherein the heparanase inhibitory substance is suramin;
(11) a wrinkle improver composed of suramin;
(12) the method of any of (1) to (6) above, wherein the substance is suramin;
(13) the orally administered pharmaceutical preparation, injection preparation or external skin preparation of any of (7) to (9), wherein the heparanase inhibitory substance is 4-(1H-benzoimidazol-2-yl)-phenylamine or a derivative thereof;
(14) a wrinkle improver composed of 4-(1H-benzoimidazol-2-yl)-phenylamine or a derivative thereof;
(15) the method of any of (1) to (6), wherein the substance is 4-(1H-benzoimidazol-2-yl)-phenylamine or a derivative thereof;
(16) the orally administered pharmaceutical preparation, injection preparation or external skin preparation of any of (7) to (9), wherein the heparanase inhibitory substance is a heparanase activity inhibitor composed of one type or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract;
(17) a wrinkle improver consisting of a heparanase activity inhibitor composed of one or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract;
(18) the method of any of (1) to (6), wherein the substance is a heparanase activity inhibitor composed of one type or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract;
(19) a method for evaluating anti-wrinkle effects, comprising contacting a test substance with skin, skin tissue or cells of a human or animal, detecting the enzyme activity, gene expression level or heparan sulfate chains of heparanase in the skin, and evaluating anti-wrinkle effects of the test substance by using changes in the enzyme activity, gene expression level or heparan sulfate chains of heparanase as an indicator;
(20) the method of (19), wherein epidermal keratinocytes are used; and,
(21) the method of (19), wherein dermal fibroblasts are used.

Heparanase present in skin can be inhibited and wrinkles, and particularly fine wrinkles, can be prevented or improved extremely effectively by applying the oral preparation, injection preparation or external skin preparation of the present invention or by the cosmetic method of the present invention. In addition, according to the evaluation method of the present invention, anti-wrinkle substances having higher effects can be specified efficiently and easily.

### BRIEF DESCRIPTION OF THE DRAWINGS

FIG. 1 indicates changes in gene expression of heparanase. Results are shown as the mean ± standard deviation (*: p<0.01).
FIG. 2 shows micrographs of immunostaining using antibodies specific to perlecan core protein and heparan sulfate chains that indicate time-course changes in decomposition of heparan sulfate chains on epidermal basement membrane by heparanase. (A)-(C): Skin immunostained with antibody specific to perlecan core protein: Day 0, Day 2, Day 7. (D)-(F): Skin immunostained with antibody specific to heparan sulfate chains: Day 0, Day 2, Day 7.
FIG. 3 indicates anti-wrinkles effects of the heparanase inhibitor, suramin (*: P<0.05, n=5 to 6).
FIG. 4 shows micrographs indicating the protective effects of suramin on epidermal basement membrane heparan sulfate chains. (A)-(D): Micrographs of immunostaining of skin with antibody specific to heparan sulfate. (E)-(H): Micrographs of immunostaining of skin coated with 1 mM suramin with antibody specific to heparan sulfate.
FIG. 5 indicates anti-wrinkle effects of the heparanase inhibitor, 1-[4-(1H-benzoimidazol-2-yl)-phenyl]-3-[4-(1H- benzoimidazol-2-yl)-phenyl]-urea (*: P<0.001, n=6).

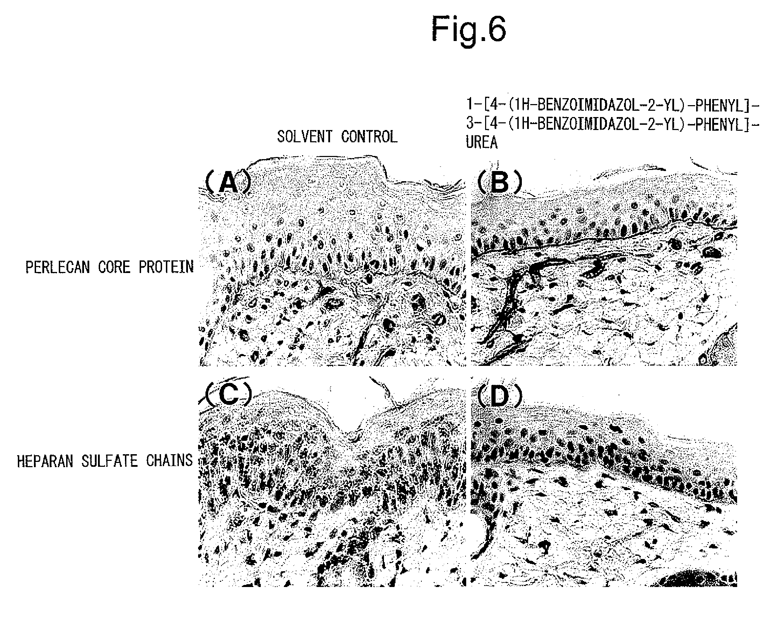

FIG. 6 shows micrographs of immunostaining with antibodies specific to perlecan core protein and heparan sulfate chains that indicate protective (decomposition inhibitory) effects on epidermal basement membrane heparan sulfate chains of 1-[4-(1H-benzoimidazol-2-yl)-phenyl]-3-[4-(1H- benzoimidazol-2-yl)-phenyl]-urea. (A): Micrograph of immunostaining of skin coated with 50% ethanol with perlecan-specific antibody. (B): Micrograph of immunostaining of skin coated with 1 mM 1-[4-(1H-benzoimidazol-2-yl)-phenyl]-3-[4-(1H- benzoimidazol-2-yl)-phenyl]-urea with perlecan-specific antibody. (C): Micrograph of immunostaining of skin coated with 50% ethanol with heparan sulfate-specific antibody. (D): Micrograph of immunostaining of skin coated with 1 mM 1-[4-(1H-benzoimidazol-2-yl)-phenyl]-3-[4-(1H-benzoimidazol-2-yl)-phenyl]-urea with heparan sulfate-specific antibody.
FIG. 7 indicates results of evaluating inhibition of heparanase activity of 4-(1H-benzoimidazol-2-yl)-phenylamine.
FIG. 8 shows micrographs of HT1080 cells indicating the results of a wound healing assay using HT1080 cells of 4-(1H-benzoimidazol-2-yl)-phenylamine. (A): Micrograph showing cells immediately after scratching. (B): Micrograph showing cells 12 hours after scratching after having replaced the medium with medium containing DMSO. (C-E): Micrographs showing cells 12 hours after scratching after having replaced the medium with medium containing 4-(1H-benzoimidazol-2-yl)- phenylamine (C: 1 µM, D: 10 µM, E: 100 µM).
FIGS. 9(A) and 9(B) show the results of screening for inhibition of heparanase activity among examples.

### BEST MODE FOR CARRYING OUT THE INVENTION

Heparanase is an enzyme present in various cells that specifically decomposes heparan sulfate chains of various heparan sulfate proteoglycans. In the skin, it is produced by, for example, epidermal keratinocytes that compose the epidermis, dermal fibroblasts and vascular endothelial cells. Production is also known to increase in various types of cancer cells, and a correlation with cancer malignancy has also been suggested. Metastasis and induction of vascular neogenesis are also known to be high as production of heparanase in cancer cells increases (see Non-Patent Document 3). In the present invention, expression and activation of heparanase was found to increase and decomposition of heparan sulfate chains was also newly discovered in a fine wrinkle model.
Moreover, heparan sulfate of basement membrane has been determined to be decomposed to a greater extent than exposed areas of skin in senile lentigo tissue. Accompanying decomposition of heparan sulfate, control of vascular endothelial cell growth factor A (VEGF-A) expressed in the epidermis is disrupted, thereby causing inflammation due to changes in dermal vessels and lymph vessels and resulting in activation of melanocytes. In addition, disruption of the control of fibroblast growth factor 7 (FGF-7) expressed in the dermis accelerates the transfer of melanosomes from melanocytes in epidermal cells. Namely, decomposition of heparan sulfate accompanying heparanase activation causes melanocytes to geometrically accumulate in keratinocytes due to acceleration of melanocyte transfer attributable to melanocyte activation and disruption of FGF-7 control caused by inflammation.

Heparan sulfate proteoglycans act by causing extracellular accumulation of heparin-binding growth factors (such as bFGF, HGF, VEGF or HB-EGF). Perlecan, which is a type of heparan sulfate proteoglycan, is also present in epidermal basement membrane located at the boundary between the epidermis and dermis, and in the skin, controls migration of growth factors between the epidermis and dermis by causing heparan sulfate-binding growth factors to bind to epidermal basement membrane.
In addition, perlecan present in epidermal basement membrane also controls the action of growth factors on epidermal basement membrane cells bound to the basement membrane, and has been determined to be essential for proper growth and differentiation of the epidermis.

Thus, decomposition of heparan sulfate chains caused by activation or increased expression of heparanase disrupts release of accumulated growth factors as well as control of growth factors between the epidermis and dermis, and the resulting disruption of control of differentiation and growth of the epidermis and hypertrophy of the dermis is considered to be an important mechanism of fine wrinkle formation. Therefore, as a result of attempting to inhibit heparanase activity in skin by externally applying suramin, which inhibits heparanase activity, it was found that decomposition of heparan sulfate chains is inhibited, and the formation of fine wrinkles in particular is suppressed.

The cosmetic method for preventing or improving wrinkles of the present invention was completed by controlling heparanase based on the finding that inhibiting heparanase, which increases accompanying disruption of the skin barrier, by inhibiting gene expression, translation, enzyme activation and activated enzymes makes it possible to inhibit wrinkle formation that occurs due to continuous disruption of the skin barrier, while the wrinkle formation preventive and improver was completed by allowing a heparanase control agent to reach the skin by a route such as oral administration, injection or external skin application.

A heparanase controlling substance, such as a substance that inhibits the activity of heparanase present in skin, a substance that inhibits its expression, a substance that inhibits its translation, or a substance that inhibits its activation, is contained as an active ingredient for the substance that prevents or improves wrinkles of the present invention.

In addition, the cosmetic method for preventing or improving wrinkles of the present invention controls heparanase in the skin by administering the aforementioned heparanase controlling substance for heparanase present in skin by a method consisting of oral administration, injection or external application.

In addition, an example of such a heparanase controlling substance is a substance such as suramin that inhibits the activity of heparanase.

The present invention also provides wrinkle improvers composed of suramin. These compounds have the action of preventing wrinkle formation or improving wrinkles that have already formed by inhibiting the activity of heparanase.

In the present description, "heparanase control" refers not only to inhibiting the enzyme activity of heparanase, but also to inhibition of gene expression and protein biosynthesis, and includes any arbitrary action that inhibits the action of heparanase in skin, such as inhibiting the activation of heparanase.

The method for evaluating anti-wrinkle effects of the present invention consists of contacting a test substance with skin, skin tissue or cells of a human or animal, and evaluating anti-wrinkle effects by using changes in heparanase enzyme activity, gene expression level or heparan sulfate chains of heparanase in the skin, tissue or cells as an indicator.

In the present description, "anti-wrinkle effects" refer to any arbitrary effects that prevent wrinkle formation or improve wrinkles that have already been formed.

In the cosmetic method for preventing or improving wrinkles of the present invention, the substance that controls heparanase can be applied in any arbitrary form provided the object of the present invention is able to be achieved, and may be applied alone or may be applied by incorporating with other arbitrary components. In the case of applying to skin, there are no limitations on the location of the skin where it is applied, and application sites include all skin on the surface of the body, including the scalp.
The method for evaluating anti-wrinkle effects of the present invention includes a step in which a test substance is contacted with skin, skin tissue, cells or enzyme of a human or animal.

There are no particular limitations on the skin of a human or animal that can be used in the present evaluation method provided the object of the present invention is able to be achieved. In the evaluation method of the present invention, it is deemed that an anti-wrinkle substance can be efficiently specified by, for example, specifying a substance that lowers increased gene expression in a fine wrinkle model or a substance that inhibits decomposition of heparan sulfate chains.

As will be described in detail in the following examples, primary evaluation of heparanase activity can be carried out by, for example, immobilizing biotinylated heparan sulfate in a 96-well plate, allowing the heparanase to act in the presence of a pharmaceutical preparation or herbal medicine, and evaluating the activity of heparanase as the amount of the decrease in biotinylated heparan sulfate by allowing peroxidase-labeled avidin to act thereon and developing the resulting color. Those pharmaceutical preparations that demonstrate heparanase activity inhibitory effects in the primary evaluation can be evaluated for reproducibility and concentration dependency with a secondary evaluation system that uses heparanase activity differing from the primary evaluation system as an indicator. Secondary evaluation can be carried out by using HT1080 cells expressing heparanase. When HT1080 cells are scratched after having been cultured to confluency, cells are known to migrate in a heparanase activity-dependent manner (see Ishida, K., et al., Mol. Cancer Ther., 2004, 3(9): 1069-1077 (Non-Patent Document 4)). Therefore, heparanase inhibitory activity can be evaluated on the basis of the degree of migration (recovery) at the scratched site by adding an evaluation reagent.

As a result, 4-(1H-benzoimidazol-2-yl)-phenylamine was found to be a compound that significantly inhibits heparanase activity. 4-(1H-benzoimidazol-2-yl)-phenylamine and derivatives thereof were completely unknown in the prior art to demonstrate heparanase activity inhibitory action and anti-aging action. Furthermore, "anti-aging" refers to preventing and improving skin wrinkles, sagging or hardening and the like as well as maintaining skin in a resilient, youthful and healthy state by inhibiting skin changes accompanying release of heparan sulfate-binding growth factors caused by decomposition of heparan sulfate of basement membrane proteoglycans attributable to aging and photoaging, and more specifically, by inhibiting abnormal epidermal differentiation, dermal vascular neogenesis, lymphatic duct dilation and elastin decomposition.

In addition, as a result of conducting further screening, herbal medicines such as valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract were found to significantly inhibit heparanase activity. These herbal medicines were also not known whatsoever in the prior art to demonstrate heparanase activity inhibitory action and anti-aging action.

Valerian extract is an extract obtained by extracting from the root or rhizomes of Valeriana fauriei, a perennial belonging to the genus Valeriana of the family Valerianaceae, or other related species. Examples of related species include V. flaccidissima and V. officinalis.

Cypress extract is an extract having for its main component hinokitiol that is obtained by extracting from the leaves, branches or wood of trees referred to as natural cypress or natural hiba belonging to the genus Chamaecyparis or Thujopsis of the family Cupressaceae. Hinokitiol has antibacterial, bactericidal, insecticidal, fungicidal and antiseptic effects. Examples of natural cypress that are used preferably include Japanese cypress (Chamaecyparis obtuse) and Taiwan cypress distributed along the central mountain range of Taiwan (Chamaecyparis obtuse var. formosana). Examples of natural hiba that are used preferably include Thujopsis delabrata and its variant, T. d. var. hondae. So-called Tsugaru hiba (also referred to as Mutsu hiba or Aomori hiba), which grows in Aomori prefecture, is well-known.

Kiwi extract is an extract obtained by extracting from the fruit of kiwi (Actinidia chinensis) belonging to the genus Actinidia of the family Actinidiaceae. In addition to vitamin C, this extract contains proteinases, amino acids, tannin, sugars and other natural plant components, and has moisturizing action, astringent action, skin softening action and antioxidative action.

Lemon extract is an extract obtained by extracting from the fruit of Citrus limon belonging to the genus Citrus of the family Rutaceae. This extract contains vitamin A, vitamin B, vitamin C, vitamin P and the like. It has whitening and anti-inflammatory effects.

Tomato extract is an extract obtained by extracting from the skin or fruit of Lycopersicon esculentum belonging to the genus Lycopersicon of the family Solanaceae. Polyphenols unique to tomatoes are contained particularly in the skin, and have anti-allergic activity.

Garlic extract is an extract obtained by extracting from the bulbs of Allium sativum or Allium scoroplasum belonging to the genus Allium of the family Liliaceae. This extract contains substances such as allicin, scordinin, enzymes and vitamins, and has fatigue recovery, stomach medicinal and intestinal regulatory effects. Allicin contained in garlic demonstrates potent bactericidal and antimicrobial activity, while scordinin has analeptic action, activates metabolism and enhances the effects of vitamin B1.

Lily extract is an extract obtained by extracting from the bulbs of Lilium candidum (also known as madonna lily) belonging to the genus Lilium of the family Liliaceae. In addition to anthocyanins and oxidases, this extract contains large amounts of starch and demonstrates moisturizing action, protective action and softening action.

Peucedanum japonicum extract is an extract obtained by extracting from the leaves or stems of Peucedanum japonicum, which is an evergreen perennial belonging to the genus Peucedanum of the family Umbelliferae. This extract contains large amounts of carotene, vitamin C and vitamin E, and has antioxidative action.

Bitter orange peel extract is an extract obtained by extracting from the skin of Citrus aurantium belonging to the genus Citrus of the family Rutaceae. The skin of the mature form of the fruit is referred to as bitter orange peel. Bitter orange peel extract contains limonene, hesperidin, oily components and the like, and has analgesic and skin function activating effects.

Sapindus mukurossi peel extract is an extract obtained by extracting from the skin of Sapindus mukorossi belonging to the genus Sapindus of the family Sapindaceae. This extract contains the natural surfactant, Mukorossi saponin, and demonstrates detergent effects as well as anti-inflammatory action and antimicrobial action.

Parsley extract is an extract obtained by extracting from the leaves of Petroselinum crispum, which is a perennial that grows in Europe belonging to the genus Petroselinum of the family Umbelliferae. Its main components include apiol and myristicin. Apiol has diuretic effects.

Jujuba fruit extract is an extract obtained by extracting from the fruit of Ziziphus jujuba var. inermis belonging to the genus Ziziphus of the family Rhamnaceae. Its main components consist of saponin, fructose and other sugars, organic acids and nucleic acrid-associated substances such as cyclic AMP. This cyclic AMP has functions such as neogenesis (regeneration) of sebaceous tissue proteins and regulation of sebum secretion.

Unshiu peel extract is a Chinese herbal medicine obtained by extracting from the skin of Citrus unshiu in Japan, or Citrus chachiensis in China, that belongs to the genus Citrus of the family Rutaceae. It contains extremely high levels of polyphenols, and demonstrates anti-inflammatory and circulation promoting actions.

Nettle extract is an extract obtained by extracting from the leaves of Urtica thunbergiana or Urtica dioica, which is a perennial that grows wild in shady areas of mountainous regions belonging to the genus Urtica of the family Urticaceae. This extract has astringent and deodorizing actions.

Each of the aforementioned plant extracts can be obtained in accordance with ordinary methods, such as by immersing or hot-refluxing the plant serving as the source of each extract with an extraction solvent followed by filtering and concentrating. Any arbitrary solvent can be used for the extraction solvent provided it is a solvent that is ordinarily used for extraction, examples of which include water, alcohols such as methanol, ethanol, propylene glycol, 1,3-butylene glycol or glycerin, water-containing alcohols, and other organic solvents such as chloroform, dichloroethane, carbon tetrachloride, acetone, ethyl acetate or hexane, and these can be used alone or in combination. An extract obtained by extracting with solvent as described above can be used as is or a concentrated extract may be removed of impurities using an adsorption method using an ion exchange resin and the like, or an extract can be used after adsorbing with a porous polymer column (such as Amberlite XAD-2) followed by eluting with methanol or ethanol and concentrating. In addition, an extract obtained by such extracting methods can be subjected to further extraction such as steam distillation or a distribution method, e.g., extraction with water and ethyl acetate, and such an extract or the like can also be used.

Each of the aforementioned extracts is highly safe and has superior heparanase activity inhibitory action. Thus, they are useful in treating, preventing or improving various symptoms, diseases or pathological conditions caused by heparanase activity. They are particularly preferably applicable to anti-aging based on inhibition of heparanase in skin. More specifically, these extracts are preferably used to prevent or improve skin wrinkles, sagging or hardening and maintain skin in a resilient, youthful state by inhibiting disruption of control of heparin-binding growth factors (HBGF) caused by decomposition of heparan sulfate chains of basement membrane proteoglycans attributable to aging or photoaging and the like, and by inhibiting skin changes accompanying the resulting release of HBGF.

In the case of incorporating the heparanase activity inhibitor of the present invention in an external skin preparation, the incorporated amount of the heparanase activity inhibitor within the total amount of the external skin preparation in terms of the dry weight (solid fraction weight) is preferably 0.0001 to 1% by weight, and particularly preferably 0.0001 to 0.2% by weight. If the incorporated amount is less than 0.0001% by weight, it becomes difficult to adequately demonstrate the effects of the present invention, while if the incorporated amount exceeds 1% by weight, significant improvement of effects is not observed and formulation becomes difficult, thereby making this undesirable.

In the case of applying the heparanase activity inhibitor of the present invention to an external skin preparation, for example, in addition to the essential ingredients described above, ingredients ordinarily used in cosmetics, pharmaceuticals and other external preparations can be suitably incorporated as necessary within a range that does not impair the effects of the present invention, examples of which include whitening agents, moisturizers, antioxidants, oily components, ultraviolet absorbers, surfactants, thickeners, alcohols, powdered components, pigments, aqueous components, water and various types of skin nutrients.

Moreover, metal ion chelating agents such as disodium edetate, trisodium edetate, sodium citrate, sodium polyphosphate, sodium metaphosphate or gluconic acid, preservatives such as methyl p-hydroxybenzoate, ethyl p-hydroxybenzoate or butyl p-hydroxybenzoate, pharmaceutical agents such as caffeine, tannin, verapamil, tranexamic acid and derivatives thereof, licorice extract, glabridin, quince fruit hot water extract, various herbal medicines, tocopherol acetate, glycyrrhetic acid and derivatives thereof or salts thereof, whitening agents such as vitamin C, magnesium ascorbyl phosphate, ascorbic acid glucoside, albutin or kojic acid, sugars such as glucose, fructose, mannose, sucrose or trehalose, or vitamin A derivatives such as retinoic acid, retinol, retinol acetate or retinol palmitate can also be suitably incorporated.

In addition, this external skin preparation can be particularly preferably applied to a wide range of cosmetics such as cosmetics applied to the skin or quasi-drugs, the drug form thereof may be any form provided it allows the preparation to be applied to the skin, and any arbitrary drug form such as a solution system, solubilized system, emulsified system, dispersed powder system, water-oil bilayer system, water-oil-powder trilayer system, ointment, beauty wash, gel or aerosol can be applied.

The dosage form is also arbitrary, and examples thereof that can be used include facial cosmetics such as beauty washes, milky lotions, creams or packs, makeup cosmetics such as foundation, lipstick or eye shadow, aromatic cosmetics and bath additives.

In addition, the external skin preparation can also be applied to a wide range of makeup cosmetics such as foundations or toiletry products such as body soaps or hand soaps. Moreover, the external skin preparation can also be applied to a wide range of quasi-drugs such as various types of ointments. The form in which the heparanase activity inhibitor of the present invention can be used is not limited by these drug forms and forms of use.

In the case of using the heparanase activity inhibitor of the present invention as a pharmaceutical preparation, the preparation can be suitably used orally or parenterally (such as intravenous administration or intraperitoneal administration). The drug form thereof is also arbitrary, and can be suitably prepared in any form according to known methods, examples of which include oral solid preparations such as tablets, granules, powders or capsules, oral liquid preparations such as internally taken liquids or syrups, and parenteral liquid preparations such as injection preparations. These pharmaceutical preparations may also suitably use ordinarily used vehicles such as binders, disintegration agents, thickeners, dispersants, reabsorption promoters, correctives, buffers, surfactants, dissolution assistants, preservatives, emulsifiers, isotonic agents, stabilizers or pH adjusters.

### Examples

Although the following provides a detailed explanation of the present invention through examples thereof, the present invention is not limited to the following examples.

### Evaluation of Anti-Wrinkle Effects of Heparanase Inhibitor, Suramin

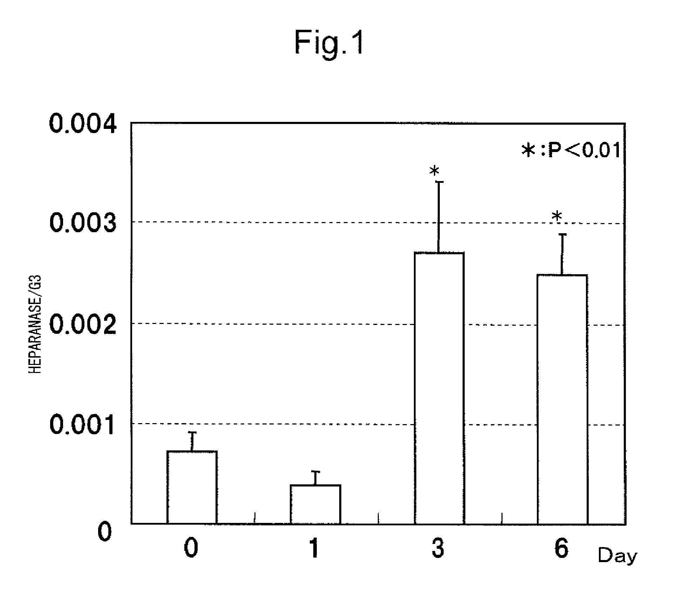

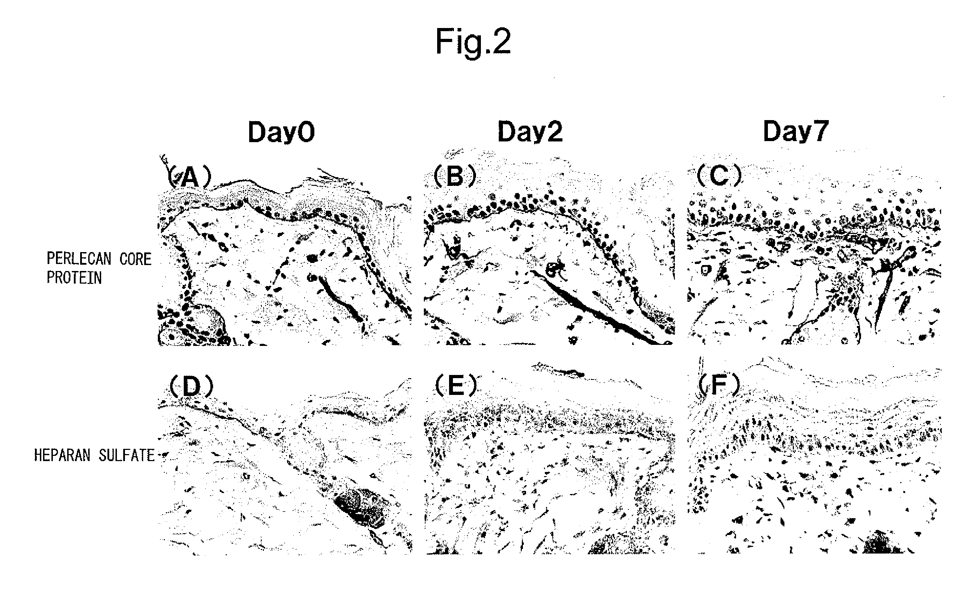

The anti-wrinkle effects of the heparanase inhibitor, suramin, were assessed using a fine wrinkle model (Non-Patent Document 2).
As shown in FIG. 1, expression of heparanase gene increased in the fine wrinkle model. In the skin on day 7, staining of heparan sulfate chains on epidermal basement membrane present at the epidermal-dermal junction nearly completely disappeared as shown in FIG. 2.

Suramin was dissolved in 50% aqueous ethanol solution to a concentration of 1 mM and applied in 100 µl aliquots to skin of the fine wrinkle model three times a week for a period of two weeks during which time fine wrinkles are formed. A 50% aqueous ethanol solution was used as a solvent control.

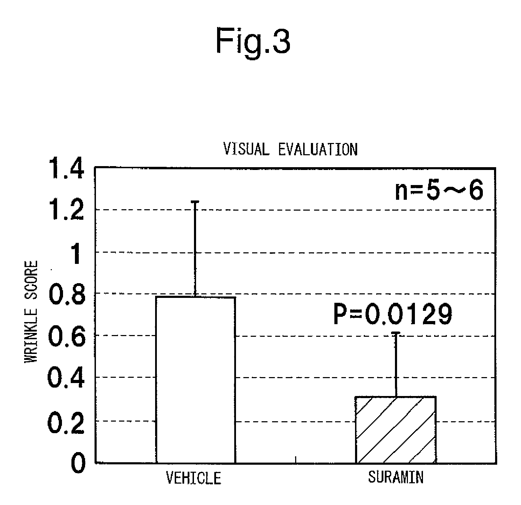

The formation of wrinkles two weeks later was scored by visual evaluation. Wrinkle formation was scored in increments of 0.5 based on evaluation criteria consisting of: no wrinkles: 0, slight wrinkles: 1, obvious wrinkles: 2 and deep wrinkles: 3. Higher scores indicate deeper wrinkles. The mean values and standard deviations were calculated for each group and the results are shown in FIG. 3. In contrast to the mean value of the scores of the solvent control being 0.78, that for suramin was 0.31, thereby indicating significant wrinkle inhibitory effects as compared with the solvent control. Namely, the application of suramin at 1 mM significant inhibited wrinkle formation as compared with 50% ethanol applied as a solvent control.

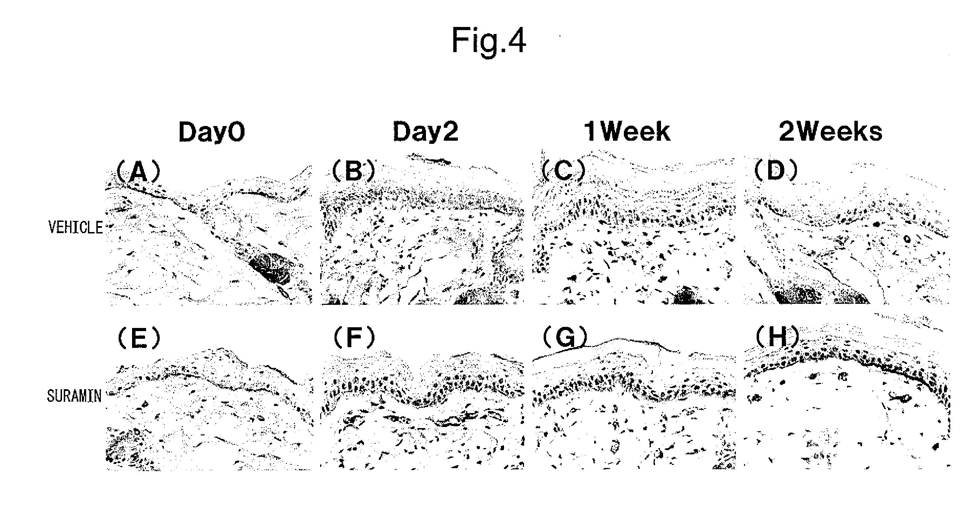

Next, heparan sulfate chains were immunostained for the skin after 2 days, 1 week and 2 weeks, and the staining results are shown in FIG. 4. Although staining of basement membrane heparan sulfate chains decreased from 2 days to 1 week in skin of the solvent control, stainability of basement membrane heparan sulfate chains was protected by application of suramin.

On the basis of these results, suramin was determined to inhibit fine wrinkle formation by inhibiting heparanase enzyme.

### Evaluation of Anti-Wrinkle Effects of Heparanase Inhibitor, 1-[4-(1H-benzoimidazol-2-yl)-phenyl]-3-[4-(1H-benzoimidazol-2-yl)-phenyl]-urea

The anti-wrinkle effects of the known specific heparanase inhibitor, 1-[4-(1H-benzoimidazol-2-yl)-phenyl]-3-[4-(1H- benzoimidazol-2-yl)-phenyl]-urea (Pan, W., et al., Bioorg. Med. Chem. Lett., 2006, (16):909-412 (Non-Patent Document 5)), were assessed.
1-[4-(1H-benzoimidazol-2-yl)-phenyl]-3-[4-(1H-benzoimidazol-2-yl)-phenyl]-urea was dissolved in 50% aqueous ethanol solution to a concentration of 1 mM and applied to the skin in 100 µl aliquots in the same manner as suramin. 50% aqueous ethanol solution was applied as a solvent control.

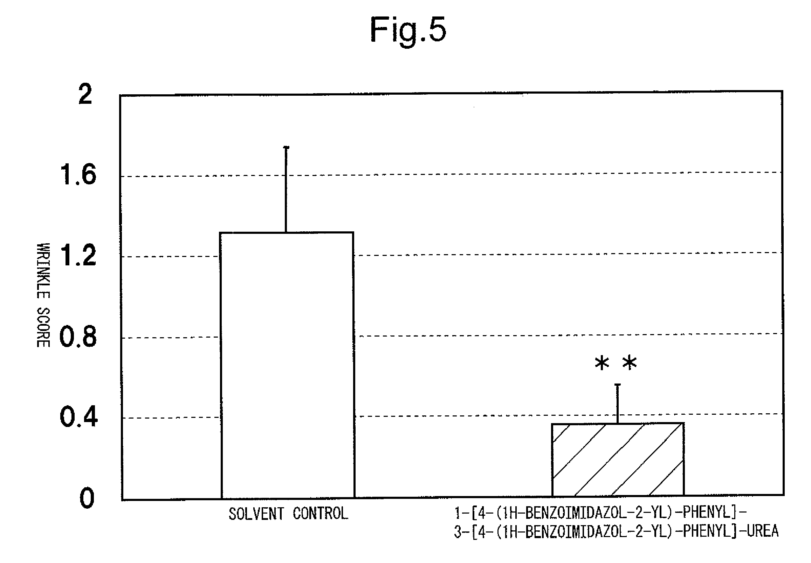

The formation of wrinkles two weeks later was scored by visual evaluation. Wrinkle formation was scored in increments of 0.5 based on evaluation criteria consisting of: no wrinkles: 0, slight wrinkles: 1, obvious wrinkles: 2 and deep wrinkles: 3. Higher scores indicate deeper wrinkles. The mean values and standard deviations were calculated for each group and the results are shown in FIG. 5. In contrast to mean value of the scores of the solvent control (50% aqueous ethanol solution) being 1.31, that for 1-[4-(1H-benzoimidazol-2-yl)- phenyl]-3-[4-(1H-benzoimidazol-2-yl)-phenyl]-urea was 0.36, and wrinkle formation was significantly inhibited.

Next, heparan sulfate chains and perlecan core protein in the skin were immunostained, and the staining results are shown in FIG. 6. Although staining of basement membrane heparan sulfate chains decreased considerably for the solvent control, stainability of basement membrane heparan sulfate chains was protected in the 1-[4-(1H-benzoimidazol-2-yl)-phenyl]- 3-[4-(1H-benzoimidazol-2-yl)-phenyl]-urea application group.

On the basis of these results, 1-[4-(1H-benzoimidazol- 2-yl)-phenyl]-3-[4-(1H-benzoimidazol-2-yl)-phenyl]-urea was determined to inhibit fine wrinkle formation by inhibiting heparanase enzyme.

### Screening for Heparanase Activity Inhibition (1) Primary Evaluation

### Evaluation Based on Heparanase Activity Inhibition

A431 cells (invasive human epithelial carcinoma cells) were cultured in DMEM medium containing 10% serum. After lysing the cultured cells with lysis buffer (50 mM Tris, 0.5% Triton X-100, 0.15 M NaCl, pH 4.5) and recovering with a scraper, the cells were pipetted and allowed to stand undisturbed on ice for 30 minutes. Insoluble matter was subsequently removed by centrifuging for 10 minutes at 10,000 rpm and the resulting supernatant was used as a cell extract. The amount of protein in the cell extract was measured with a BCA Protein Assay Kit (Pierce, CA46141).

The cell extract was diluted to a prescribed concentration with assay buffer (50 mM HEPES, 50 mM CH₃COONa, 150 mM NaCl, 9 mM CaCl₂, 0.1% BSA) followed by the addition of 4-(1H-benzoimidazol-2-yl)-phenylamine, mixing, and inoculation into a biotinylated heparan sulfate-immobilized plate at 100 µL/well. After allowing to react for 2 hours at 37°C, the plate was washed three times with PBS-T and HRP-avidin diluted by a factor of 10,000 (Vector, A-2004)/PBS-T was inoculated at 100 µL/well followed by allowing to react for 1 hour at 37°C. After again washing the plate three times with PBS-T, TMB reagent (Bio-Rad, 172-1066) was inoculated at 100 µL/well, and after stopping the reaction with 1 N H₂SO₄, optical density was measured at 475 nm (see Published Japanese Translation of PCT Application No. 2003-502054 (Patent Document 3)).
Heparanase activity was calculated from a calibration curve of the A431 cell extract, and inhibition rate (%) was indicated as a relative value to a reference (control) to which the cell extract was not added.

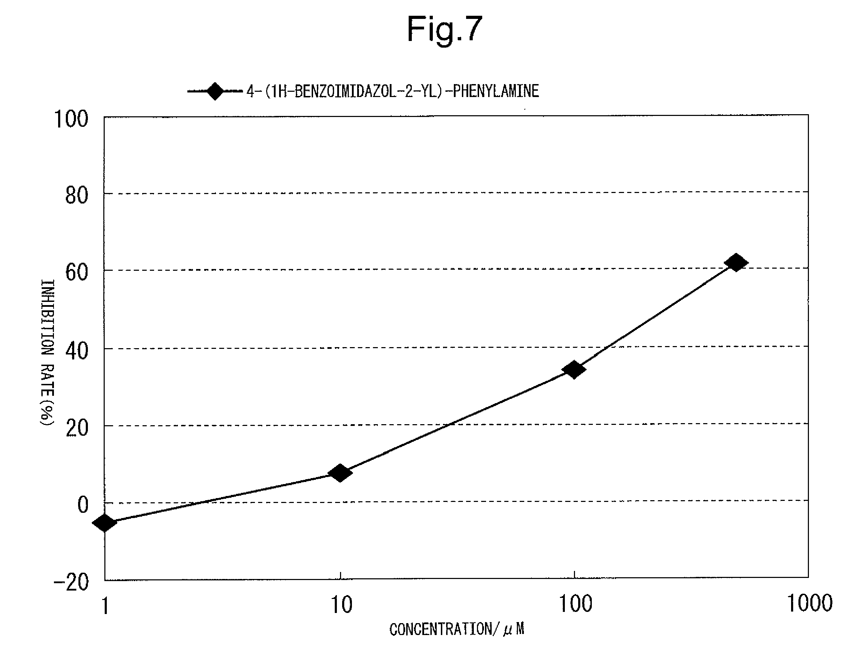

As a result, 4-(1H-benzoimidazol-2-yl)-phenylamine was determined to concentration-dependently effectively inhibit heparanase activity. The results are shown in FIG. 7. DMSO was allowed to act as a control instead of a candidate drug.
IC50 = 256 µM

### Secondary Evaluation

### Wound Healing Assay Using HT1080 Cells

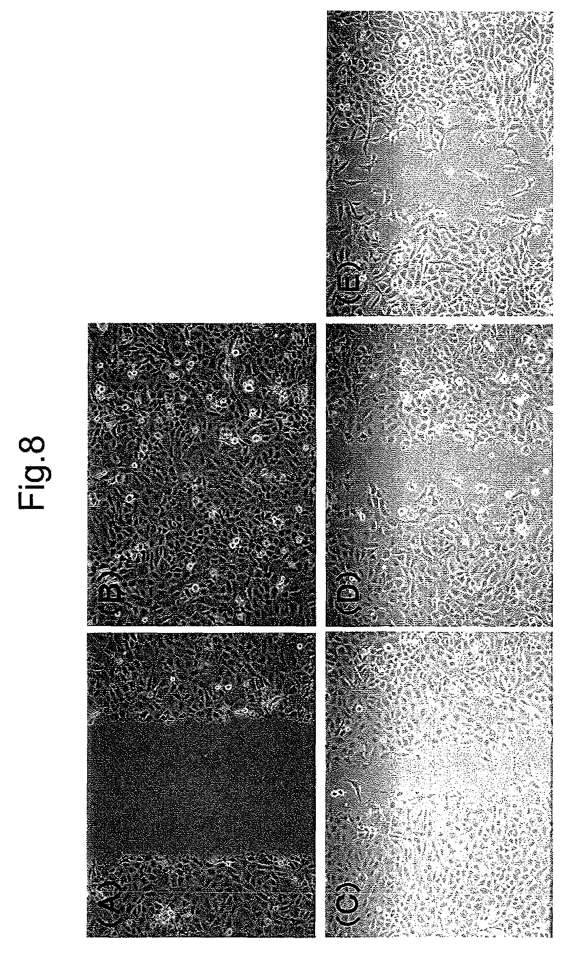

HT1080 cells were inoculated into a 6-well plate at 500,000 cells/well, and the medium was replaced with that containing each drug 24 hours later followed by scratching in the vertical direction with a 1000 µL tip. Micrographs were taken 6 hours later and changes at the scratched site were observed. As a result, cell migration activity decreased in a concentration-dependent manner at the site where 4-(1H-benzoimidazol-2-yl)-phenylamine was added, thereby clearly demonstrating that heparanase activity is inhibited. The results are shown in FIG. 8. DMSO was allowed to act as a control instead of the candidate drugs.

### Screening for Heparanase Activity Inhibition (2)

### 1. Sample Preparation

### (1) Plant Extracts

As shown in Table 1, each plant was immersed in the solvent shown in the table for 1 week at room temperature to obtain extracts. The extracts were concentrated to obtain each plant extract.

**[Table 1]**

| Plant Name | Site | Amt. of plant used (g) | Solvent | Amt. of solvent used (mL) | Plant extract yield (g) |
|---|---|---|---|---|---|
| Valeriana fauriei | Rhizome | 100 | Water | 1000 | Valerian extract (17.2 g) |
| Chamaecyparis obtuse | Trunk | 500 | Water | 5000 | Cypress extract (1.3 g) |
| Actinidia chinensis | Fruit | 100 | 30% aqueous ethanol | 1000 | Kiwi extract (10.5 g) |
| Citrus limon | Fruit | 200 | 30% aqueous ethanol | 1000 | Lemon extract (9.8 g) |
| Lycopersicon esculentum | Fruit | 200 | Water | 1000 | Tomato extract (5.0 g) |
| Allium sativum | Bulb | 100 | 30% aqueous ethanol | 1000 | Garlic extract (11.0 g) |
| Lilium candidum | Bulb | 100 | 80% aqueous ethanol | 1000 | Lily extract (6.9 g) |
| Peucedanum japonicum | Leaf and stem | 100 | Methanol | 1000 | Peucedanum japonicum extract (13.2 g) |
| Citrus aurantium | Skin | 100 | 30% aqueous ethanol | 1000 | Bitter orange peel extract (38.0 g) |
| Sapindus mukorossi | Skin | 100 | 80% aqueous ethanol | 1000 | Sapindus mukurossi peel extract (7.3 g) |
| Petroselinum crispum | Leaf | 100 | 50% aqueous ethanol | 1000 | Parsley extract (1.6 g) |
| Ziziphus jujuba | Fruit | 100 | 10% aqueous ethanol | 1000 | Jujuba fruit extract (42.0 g) |
| Citrus unshiu | Skin | 100 | Water | 1000 | Unshiu peel extract (12.5 g) |
| Urtica thunbergiana | Leaf | 100 | 80% aqueous ethanol | 1000 | Nettle extract (8.4 g) |

### (2) Sample Solution

Each of the plant extracts was dissolved in dimethylsulfoxide (DMSO) to a concentration of 1% by weight to prepare plant extract-containing solutions.

Each of these plant extract-containing solutions was diluted with assay buffer (pH 7.4 0.1 M Tris containing 0.4 M NaCl and 10 mM CaCl₂) to concentrations of 0.05 v/v% 0.5 v/v% and 5 v/v%, and the resulting diluted solutions were used as sample solutions in the following experiments.

### 2. Method Used to Evaluate Heparanase Inhibitory Effects and Results

The plant extracts were evaluated for heparanase inhibitory effects in accordance with the aforementioned primary and secondary evaluation methods using sample solutions of the plant extracts instead of 4-(1H-benzoimidazol-2-yl)-phenylamine.

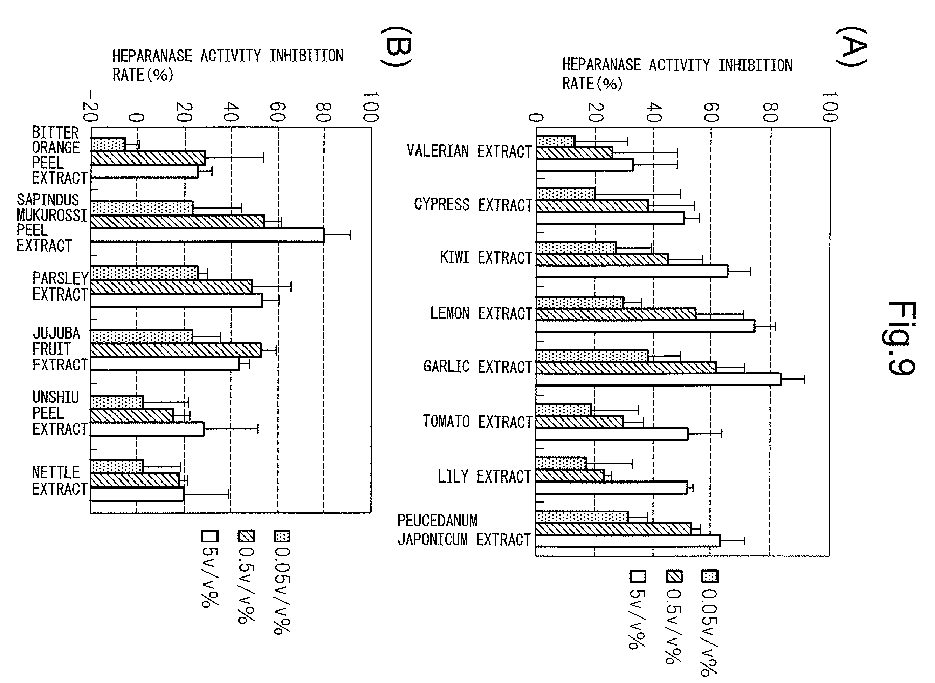

The results are shown in FIGS. 9(A) and 9(B).

As is clear from the results shown FIGS. 9(A) and 9(B), the valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract were confirmed to effectively inhibit heparanase activity.

The following further indicates formulation examples. Furthermore, in the following examples, "POE" refers to polyoxyethylene.

| (Formulation Example 1: Cream) | | |
|---|---|---|
| (Incorporated Components) | | (wt%) |
| (1) | Stearic acid | 3.0 |
| (2) | Stearyl alcohol | 5.0 |
| (3) | Isopropyl myristate | 18.0 |
| (4) | Glycerin monostearic acid ester | 3.0 |
| (5) | Propylene glycol | 10.0 |
| (6) | Present heparanase activity inhibitor (valerian extract (50% aqueous ethanol extract, as solid fraction)) | 1.0 |
| (7) | Potassium hydroxide | 0.2 |
| (8) | Sodium hydrogen sulfite | 0.01 |
| (9) | Preservative | As suitable |
| (10) | Fragrance | As suitable |
| (11) | Ion exchange water | Balance |

### (Production Method)

Components (5) to (7) are added to component (11) followed by heating and holding at 70°C (aqueous phase). Components (1) to (4) and (8) to (10) are mixed followed by heating, melting and holding at 70°C (oily phase). The oily phase is gradually added to the aqueous phase and once the entire oily phase has finished being added, the mixture is briefly held at that temperature and allowed to react. Subsequently, the mixture is uniformly emulsified with a homomixer and cooled to 30°C while stirring well.

| (Formulation Example 2: Cream) | | |
|---|---|---|
| (Incorporated Components) | | (wt%) |
| (1) | Stearic acid | 2.0 |
| (2) | Stearyl alcohol | 7.0 |
| (3) | Hydrogenated lanolin | 3.0 |
| (4) | Squalane | 4.0 |
| (5) | 2-octyldodecyl alcohol | 6.0 |
| (6) | POE (25 mol) cetyl alcohol ether | 3.0 |
| (7) | Glycerin monostearic acid ester | 2.0 |
| (8) | Propylene glycol | 6.0 |
| (9) | Present heparanase activity inhibitor (cypress extract (70% aqueous ethanol extract, as solid fraction)) | 0.2 |
| (10) | Sodium hydrogen sulfite | 0.03 |
| (11) | Ethyl p-hydroxybenzoate | 0.3 |
| (12) | Fragrance | As suitable |
| (13) | Ion exchange water | Balance |

### (Production Method)

Component (8) is added to component (13) followed by heating and holding at 70°C (aqueous phase). Components (1) to (7) and (9) to (12) are mixed followed by heating, melting and holding at 70°C (oily phase). The oily phase is gradually added to the aqueous phase and preliminarily emulsified, and after uniformly emulsifying with a homomixer, the mixture is cooled to 30°C while stirring well.

| (Formulation Example 3: Cream) | | |
|---|---|---|
| (Incorporated Components) | | (wt%) |
| (1) | Solid paraffin | 5.0 |
| (2) | Beeswax | 10.0 |
| (3) | Vaseline | 15.0 |
| (4) | Liquid paraffin | 41.0 |
| (5) | Glycerin monostearic acid ester | 2.0 |
| (6) | POE (20 mol) sorbitan monolauric acid ester | 2.0 |
| (7) | Powdered soap | 0.1 |
| (8) | Borax | 0.2 |
| (9) | Present heparanase activity inhibitor (kiwi extract (aqueous ethanol extract, as solid fraction)) | 0.5 |
| (10) | Sodium hydrogen sulfite | 0.03 |
| (11) | Ethyl p-hydroxybenzoate | 0.3 |
| (12) | Fragrance | As suitable |
| (13) | Ion exchange water | Balance |

### (Production Method)

Components (7) and (8) are added to component (13) followed by heating, melting and holding at 70°C (aqueous phase). Components (1) to (6) and (9) to (12) are mixed followed by heating, melting and holding at 70°C (oily phase). The oily is gradually added to the aqueous phase while stirring and allowed to react. Following completion of the reaction, the mixture is uniformly emulsified with a homomixer and then cooled to 30°C while stirring well following emulsification.

| (Formulation Example 4: Milky Lotion) | | |
|---|---|---|
| (Incorporated Components) | | (wt%) |
| (1) | Stearic acid | 2.5 |
| (2) | Cetyl alcohol | 1.5 |
| (3) | Vaseline | 5.0 |
| (4) | Liquid paraffin | 10.0 |
| (5) | POE (10 mol) monooleic acid ester | 2.0 |
| (6) | Polyethylene glycol 1500 | 3.0 |
| (7) | Triethanolamine | 1.0 |
| (8) | Carboxyvinyl polymer | 0.05 |
| (9) | Present heparanase activity inhibitor (lemon extract (50% aqueous 1,3-butylene glycol extract, as solid fraction)) | 0.03 |
| (10) | Sodium hydrogen sulfite | 0.01 |
| (11) | Ethyl p-hydroxybenzoate | 0.3 |
| (12) | Fragrance | As suitable |
| (13) | Ion exchange water | Balance |

### (Production Method)

A small amount of component (13) is dissolved in component (8) (A phase). Components (6) and (7) are added to the remainder of component (13) followed by heating, melting and holding at 70°C (aqueous phase). Components (1) to (5) and (9) to (12) are mixed followed by heating, melting and holding at 70°C (oily phase). The oily phase is added to the aqueous phase and preliminarily emulsified followed by adding the A phase, uniformly emulsifying with a homomixer and cooling to 30°C while stirring well following emulsification.

| (Formulation Example 5: Milky Lotion) | | |
|---|---|---|
| (Incorporated Components) | | (wt%) |
| (1) | Microcrystalline wax | 1.0 |
| (2) | Beeswax | 2.0 |
| (3) | Lanolin | 20.0 |
| (4) | Liquid paraffin | 10.0 |
| (5) | Squalane | 5.0 |
| (6) | Sorbitan sesquioleic acid ester | 4.0 |
| (7) | POE (20 mol) sorbitan monooleic acid ester | 1.0 |
| (8) | Propylene glycol | 7.0 |
| (9) | Present heparanase activity inhibitor (tomato extract (70% aqueous 1,3-butylene glycol extract, as solid fraction)) | 0.2 |
| (10) | Sodium hydrogen sulfite | 0.01 |
| (11) | Ethyl p-hydroxybenzoate | 0.3 |
| (12) | Fragrance | As suitable |
| (13) | Ion exchange water | Balance |

### (Production Method)

Component (8) is added to component (13) followed by heating and holding at 70°C (aqueous phase). Components (1) to (7) and (9) to (12) are mixed followed by heating, melting and holding at 70°C (oily phase). The aqueous phase is gradually added to the oily phase while stirring followed by uniformly emulsifying with a homomixer. The mixture is cooled to 30°C while stirring well following emulsification.

| (Formulation Example 6: Gel) | | |
|---|---|---|
| (Incorporated Components) | | (wt%) |
| (1) | 95% ethanol | 10.0 |
| (2) | Dipropylene glycol | 15.0 |
| (3) | POE (50 mol) oleyl alcohol ether | 2.0 |
| (4) | Carboxyvinyl polymer | 1.0 |
| (5) | Sodium hydroxide | 0.15 |
| (6) | L-arginine | 0.1 |
| (7) | Present heparanase activity inhibitor (garlic extract (50% aqueous ethanol extract, as solid fraction)) | 1.0 |
| (8) | Sodium 2-hydroxy-4-methoxybenzophenone sulfonate | 0.05 |
| (9) | Ethylenediamine tetraacetate·3Na·2H₂O | 0.05 |
| (10) | Methyl p-hydroxybenzoate | 0.2 |
| (11) | Fragrance | As suitable |
| (12) | Ion exchange water | Balance |

### (Production Method)

Component (4) is uniformly dissolved in component (12) (aqueous phase). On the other hand, components (7) and (3) are dissolved in component (1) (alcohol phase). The alcohol phase is then added to the aqueous phase. Next, components (2) and (8) to (11) are added thereto followed by neutralizing with components (5) and (6) and thickening.

| (Formulation Example 7: Beauty Essence) | | |
|---|---|---|
| (Incorporated Components) | | (wt%) |
| (A | Phase) | |
| (1) | 95% ethanol | 10.0 |
| (2) | POE (20 mol) octyldodecanol | 1.0 |
| (3) | Pantothenyl ethyl ether | 0.1 |
| (4) | Present heparanase activity inhibitor (lily extract (50% aqueous 1,3-butylene glycol extract, as solid fraction)) | 0.0225 |
| (5) | Methyl p-hydroxybenzoate | 0.15 |
| (B | Phase) | |
| (6) | Potassium hydroxide | 0.1 |
| (C | Phase) | |
| (7) | Glycerin | 5.0 |
| (8) | Dipropylene glycol | 10.0 |
| (9) | Sodium hydrogen sulfite | 0.03 |
| (10) | Carboxyvinyl polymer | 0.2 |
| (11) | Purified water | Balance |

### (Production Method)

Phases A and C are each uniformly dissolved followed by adding phase A to phase C and solubilizing. Next, phase B is added followed by filling.

| (Formulation Example 8: Pack) | | |
|---|---|---|
| (Incorporated Components) | | (wt%) |
| (A Phase) | | |
| (1) | Dipropylene glycol | 5.0 |
| (2) | POE (60 mol) hydrogenated castor oil | 5.0 |
| (B Phase) | | |
| (3) | Present heparanase activity inhibitor (Peucedanum japonicum extract (70% aqueous ethanol extract, as solid fraction)) | 0.0005 |
| (4) | Olive oil | 5.0 |
| (5) | Tocopherol acetate | 0.2 |
| (6) | Ethyl p-hydroxybenzoate | 0.2 |
| (7) | Fragrance | 0.2 |
| (C Phase) | | |
| (8) | Sodium hydrogen sulfite | 0.03 |
| (9) | Polyvinyl alcohol (degree of saponification: 90, degree of polymerization: 2,000) | 13.0 |
| (10) | thanol | 7.0 |
| (11) | Purified water | Balance |

### (Production Method)

Phases A, B and C are each uniformly dissolved followed by adding phase B to phase A and solubilizing. Next, phase C is added thereto followed by filling.

| (Formulation Example 9: Solid Foundation) | | |
|---|---|---|
| (Incorporated Components) | | (wt%) |
| (1) | Talc | 43.1 |
| (2) | Kaolin | 15.0 |
| (3) | Sericite | 10.0 |
| (4) | Zinc oxide | 7.0 |
| (5) | Titanium dioxide | 3.8 |
| (6) | Yellow iron oxide | 2.9 |
| (7) | Black iron oxide | 0.2 |
| (8) | Squalane | 8.0 |
| (9) | Isostearic acid | 4.0 |
| (10) | POE sorbitan monooleate | 3.0 |
| (11) | Isocetyl octanoate | 2.0 |
| (12) | Present heparanase activity inhibitor (bitter orange peel extract (ethanol extract, as solid fraction)) | 0.00001 |
| (13) | Preservative | As suitable |
| (14) | Fragrance | As suitable |

### (Production Method)

The powdered components of (1) to (7) are mixed well with a blender followed by the addition of the oily components of (8) to (11) and components (12), (13) and (14) thereto followed by kneading well, filling into containers and molding.

| (Formulation Example 10: Emulsified Foundation | | |
|---|---|---|
| (Cream Type) | | |
| (Incorporated Components) | | (wt%) |
| (Powdered Components) | | |
| (1) | Titanium dioxide | 10.3 |
| (2) | Sericite | 5.4 |
| (3) | Kaolin | 3.0 |
| (4) | Yellow iron oxide | 0.8 |
| (5) | Red iron oxide | 0.3 |
| (6) | Black iron oxide | 0.2 |
| (Oily Phase) | | |
| (7) | Decamethylcyclopentasiloxane | 11.5 |
| (8) | Liquid paraffin | 4.5 |
| (9) | POE-modified dimethylpolysiloxane | 4.0 |
| (Aqueous Phase) | | |
| (10) | Purified water | 46.5 |
| (11) | 1,3-butylene glycol | 4.5 |
| (12) | Present heparanase activity inhibitor (Sapindus mukurossi peel extract (30% aqueous 1,3-butylene glycol extract, as solid fraction)) | 0.0025 |
| (13) | Sorbitan sesquioleic acid ester | 3.0 |
| (14) | Preservative | As suitable |
| (15) | Fragrance | As suitable |

### (Production Method)

The aqueous phase is heated and stirred followed by adding to the adequately mixed and crushed powder components and processing with a homomixer. The heated and mixed oily phase is then added followed by processing with a homomixer, adding the fragrance while stirring and cooling to room temperature.

| (Formulation Example 11: Milky Lotion) | | |
|---|---|---|
| (Incorporated Components) | | (wt%) |
| (1) | Glycerin diisostearate | 15.0 |
| (2) | Squalane | 2.0 |
| (3) | Dimethicone | 2.0 |
| (4) | Stearyl alcohol | 3.0 |
| (5) | Present heparanase activity inhibitor (parsley extract (90% aqueous ethanol extract, as solid fraction)) | 1.0 |
| (6) | Sodium methyl stearoyl taurate | 1.0 |
| (7) | POE (20 mol) behenyl ether | 0.5 |
| (8) | Glycerin | 5.0 |
| (9) | 1,3-butylene glycol | 5.0 |
| (10) | Carboxyvinyl polymer | 0.2 |
| (11) | Preservative | As suitable |
| (12) | Purified water | Balance |

### (Production Method)

A uniform mixture of components (8) to (12) is heated to 60°C followed by adding a mixture of components (1) to (7) at 70°C thereto while stirring, uniformly dispersing and cooling to 30°C to obtain a milky lotion.

| (Formulation Example 12: Beauty Wash) | | |
|---|---|---|
| (Incorporated Components) | | (wt%) |
| (1) | Ethanol | 5.0 |
| (2) | Glycerin | 0.5 |
| (3) | Dipropylene glycol | 2.0 |
| (4) | 1,3-butylene glycol | 5.5 |
| (5) | Citric acid | 0.02 |
| (6) | Sodium citrate | 0.08 |
| (7) | Sodium hexametaphosphate | 0.03 |
| (8) | Hydroxypropyl-β-cyclodextrin | 0.1 |
| (9) | Present heparanase activity inhibitor (jujuba fruit extract (70% aqueous 1,3-butylene glycol extract, as solid fraction)) | 0.015 |
| (10) | Lavender oil | 0.1 |
| (11) | Sodium alginate | 0.001 |
| (12) | Purified water | Balance |

### (Production Method)

Each of the above components is mixed and dissolved in accordance with ordinary methods to obtain a beauty wash.

| (Formulation Example 13: Milky Lotion) | | |
|---|---|---|
| (Incorporated Components) | | (wt%) |
| (1) | Dimethylpolysiloxane | 3.0 |
| (2) | Decamethylcyclopentasiloxane | 4.0 |
| (3) | Ethanol | 5.0 |
| (4) | Glycerin | 6.0 |
| (5) | 1,3-butylene glycol | 5.0 |
| (6) | POE methylglucoside | 3.0 |
| (7) | Squalane | 2.0 |
| (8) | Potassium hydroxide | 0.1 |
| (9) | Sodium hexametaphosphate | 0.05 |
| (10) | Present heparanase activity inhibitor (unshiu peel extract (70% aqueous ethanol extract, as solid fraction)) | 0.0002 |
| (11) | Xanthan gum | 0.3 |
| (12) | Carboxyvinyl polymer | 0.1 |
| (13) | Acrylic acid-alkyl methacrylate copolymer | 0.1 |
| (14) | Methyl p-hydroxybenzoate | As suitable |
| (15) | Fragrance | As suitable |
| (16) | Purified water | Balance |

### (Production Method)

Components (9), (12) and (13) are added to component (16) and dissolved followed by mixing in component (10) and components (4) to (6). A solution obtained by adding components (14), (11) and (15) to component (3) and dissolving is then mixed in followed by the addition of a mixture of components (1), (2) and (7), emulsifying, neutralizing with component (8) and thickening.

| (Formulation Example 14: Milky Lotion) | | |
|---|---|---|
| (Incorporated Components) | | (wt%) |
| (1) | Vaseline | 1.0 |
| (2) | Dimethylpolysiloxane | 3.0 |
| (3) | Methylphenylpolysiloxane | 3.0 |
| (4) | Stearyl alcohol | 0.5 |
| (5) | Glycerin | 7.0 |
| (6) | Dipropylene glycol | 3.0 |
| (7) | 1,3-butylene glycol | 7.0 |
| (8) | Squalane | 1.0 |
| (9) | Isostearic acid | 0.5 |
| (10) | Stearic acid | 0.5 |
| (11) | Glycerin polyoxyethylene monostearate | 1.0 |
| (12) | Glycerin monostearate | 2.0 |
| (13) | Potassium hydroxide | 0.05 |
| (14) | Present heparanase activity inhibitor (nettle extract (ethanol extract, as solid fraction)) | 1.0 |
| (15) | Trisodium EDTA | 0.05 |
| (16) | Carboxyvinyl polymer | 0.1 |
| (17) | Phenoxyethanol | As suitable |
| (18) | Fragrance | As suitable |
| (19) | Purified water | Balance |

### (Production Method)

Components (6), (7) and (13) are added to component (19) followed by heating and holding at 70°C (aqueous phase). On the other hand, components (1) to (4), (8) to (12) and (17) are mixed, heated, melted and held at 70°C (oily phase). The aqueous phase is gradually added to the oily phase while stirring followed by the addition of components (15), (16), (18), (5) and (14), uniformly emulsifying with a homomixer and cooling to 30°C while stirring well following emulsification.

## Claims

1. A cosmetic method for preventing or improving wrinkles, **characterized by** controlling the action of heparanase present in skin.

2. A cosmetic method for preventing or improving wrinkles, **characterized by** applying a substance that inhibits gene expression as a method for controlling the action of heparanase present in skin.

3. A cosmetic method for preventing or improving wrinkles, **characterized by** applying a substance that inhibits gene translation as a method for controlling the action of heparanase present in skin.

4. A cosmetic method for preventing or improving wrinkles, **characterized by** applying a substance that inhibits enzyme activity as a method for controlling the action of heparanase present in skin.

5. A cosmetic method for preventing or improving wrinkles, **characterized by** applying a substance that inhibits enzyme activation as a method for controlling the action of heparanase present in skin.

6. A cosmetic method for preventing or improving wrinkles, **characterized by** administering a substance that inhibits the action of heparanase present in skin by a method such as oral administration, injection or external application.

7. An orally administered pharmaceutical preparation for preventing or improving wrinkles, comprising a substance that controls the action of heparanase present in skin as an active ingredient thereof.

8. An injection preparation for preventing or improving wrinkles, comprising a substance that controls the action of heparanase in skin as an active ingredient thereof.

9. An external skin preparation for preventing or improving wrinkles, comprising a substance that controls the action of heparanase in skin as an active ingredient thereof.

10. The orally administered pharmaceutical preparation, injection preparation or external skin preparation according to any of claims 7 to 9, wherein the heparanase inhibitory substance is suramin.

11. A wrinkle improver composed of suramin.

12. The method according to any of claims 1 to 6, wherein the substance is suramin.

13. The orally administered pharmaceutical preparation, injection preparation or external skin preparation according to any of claims 7 to 9, wherein the heparanase inhibitory substance is 4-(1H-benzoimidazol-2-yl)-phenylamine or a derivative thereof.

14. A wrinkle improver composed of 4-(1H-benzoimidazol-2-yl)-phenylamine or a derivative thereof.

15. The method according to any of claims 1 to 6, wherein the substance is 4-(1H-benzoimidazol-2-yl)-phenylamine or a derivative thereof.

16. The orally administered pharmaceutical preparation, injection preparation or external skin preparation according to any of claims 7 to 9, wherein the heparanase inhibitory substance is a heparanase activity inhibitor composed of one type or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract.

17. A wrinkle improver composed of a heparanase activity inhibitor composed of one or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract.

18. The method according to any of claims 1 to 6, wherein the substance is a heparanase activity inhibitor composed of one type or two or more types selected from the group consisting of valerian extract, cypress extract, kiwi extract, lemon extract, tomato extract, garlic extract, lily extract, Peucedanum japonicum extract, bitter orange peel extract, Sapindus mukurossi peel extract, parsley extract, jujuba fruit extract, unshiu peel extract and nettle extract.

19. A method for evaluating anti-wrinkle effects, comprising contacting a test substance with skin, skin tissue or cells of a human or animal, detecting enzyme activity, gene expression level or heparan sulfate chains of heparanase in the skin, and evaluating anti-wrinkle effects of the test substance by using changes in the enzyme activity, gene expression level or heparan sulfate chains of heparanase as an indicator.

20. The method according to claim 19, wherein epidermal keratinocytes are used.

21. The method according to claim 19, wherein dermal fibroblasts are used.
